# EUROPEAN PATENT SPECIFICATION

(11) **EP 3 562 933 B1**
(45) Date of publication and mention of the grant of the patent: **18.08.2021**
(21) Application number: 17823122.1
(22) Date of filing: 20.12.2017
(51) Int. Cl.: C12M 1/26, C12N 15/10, C12M 1/00, G01N 35/10, G01N 1/28, G01N 21/03, G01N 21/64, G01N 1/02, B01L 3/02, G01N 1/04, G01N 1/40

(54) **METHOD FOR THE ISOLATION AND TREATMENT OF PARTICULATE TARGETS**
VERFAHREN ZUR ISOLIERUNG UND BEHANDLUNG VON PARTIKELFÖRMIGEN TARGETS
PROCÉDÉ POUR L'ISOLEMENT ET LE TRAITEMENT DE CIBLES PARTICULAIRES

(30) Priority: 30.12.2016 EP 16207486
(43) Date of publication of application: 06.11.2019
(73) Proprietor: Molecular Machines & Industries AG, 8152 Glattbrugg (CH)
(72) Inventor: SEEGER, Stefan, 8702 Zollikon (CH); NIEHREN, Stefan, 85250 Altomünster (DE)
(74) Representative: Schmitz, Joseph
(86) International application number: PCT/EP2017/083872
(87) International publication number: WO 2018/122071

(56) References cited:
- WO-A1-2015/048009
- US-A1- 2005 106 718
- ANIRUDDH SARKAR ET AL: "Microfluidic probe for single-cell analysis in adherent tissue culture", NATURE COMMUNICATIONS, vol. 5, 5 March 2014 (2014-03-05), XP055382538, United Kingdom ISSN: 2041-1723, DOI: 10.1038/ncomms4421
- Dirk Ehlers: "Catalog 2015", , 2015, pages 1-43, XP055758319, Hamburg Retrieved from the Internet: URL:https://www.eppendorf.com/uploads/medi a/Katalog_2015_Int-_02.pdf [retrieved on 2020-12-09]

## Description

### TECHNICAL FIELD

The present invention relates to a method of isolating and treating a particulate target located on a substrate using a capillary cell sorting apparatus.

### STATE OF THE ART

Isolated cells from tumours, endothelial tissue, histocytes, stem cells, and other materials are a prerequisite for analysis and patient profiling in medical diagnostic applications or life science research. However, current methods of isolation of single particulate targets such as cells correspond more often to enrichment methods rather than a true isolation of single cells. For instance, filters designed to isolate epithelial tumour cells such as circulating cancer cells (CTCs) by size from bodily fluids such as blood are commercialized by Rarecells SAS and in fact rather enrich such cells. This means that the plurality of cells trapped in the filters are neither identical with respect to the cell type nor genetically speaking. It is therefore not possible to analyse the collected cells in bulk and individual cells must therefore be individually isolated in order to determine their genotype.

It is further possible to isolate individual cells or cell clusters from a tissue sample by laser microdissection methods, of which multiple variations exist. For example, WO2006031574 A1 describes the use of a specific laser based technique, called laser capture microdissection, in which a small cell area can be extracted from biological tissue samples by means of an IR-laser induced transfer utilising a thermoplastic membrane. Another variation, described in EP 0 879 408 B1 consists in a technique called laser catapult technology. First a laser is used to cut around an area of interest such as a cell in a tissue sample and second the area is catapulted into a collection vessel by using laser pulses. DE 10003588 C1 discloses a laser microdissection technology in which in a first step a laser is used for excising cells from a tissue sample on a membrane slide and isolate the cell in a second step by adhering the cut area onto an adhering cap, and subsequently, a lysis buffer is used to extract cellular components like DNA or RNA for further genetic analysis.

All of the above methods require two discrete steps: A first step of selecting and isolating the target cell from its environment and/or substrate and a subsequent second step of transferring the target cell to a separate vessel where then a treatment liquid such as for example a lysis buffer is added to the previously isolated target cell. Such methods are labour intensive, even if automated, require the use of additional containers and materials such as transfer foils and testing tubes.

While the excision of the cells from tissue samples has been mastered using laser microdissection, such methods are unsuited for isolating adherent cells, in particular cultured cells, from the bottom of a cell culture container.

It is thus desirable to provide a method, as well as a suited apparatus, by which the single particulate targets, such as for example cells, can be isolated and treated in a more effective and simple manner such as to streamline the handling of such single particulate targets.

US2005/106718 A1 discloses a pipette tip having a blade formed along a forward end of the tip that functions as a cell scraper for mechanically disrupting monolayers of cultured cells in culture plates. The device is constructed to allow the tip to be articulated so that the blade contacts the culture plate's surface parallel to, or on a slanting angle relative to, the horizontal surface of the plate, when the pipette is held vertically. The bent tip may be used to pipette the solution, in addition to scraping the cells. After scraping, the tip of the invention can be used to collect the cell suspension from the culture dish.

Aniruddh Sarkar et al: "Microfluidic probe for single-cell analysis in adherent tissue culture", Nat. Commun. Vol. 5, 3421 (2014), XP055382538, https://doi.ora/10.1038/ncomms4421 discloses the isolation of single cells and the use of microfluidics where the lysis is carried out on the surface where the cells were adherent via hydrodynamic confinement of lysis buffer. The thus formed single-cell lysate is then pulled into a separate and different container.

WO2015/048009 A1 discloses a system and method for in situ laser lysis for analysis of live tissue at the single cell resolution.

### SUMMARY OF THE INVENTION

The method of the present invention overcome the above problems by providing a method in which the isolation and treatment of a target particulate such as a cell are carried out concurrently, by in essence drawing the particulate target into a capillary filled with a treatment liquid and carrying out the treatment inside the capillary instead of transferring the particulate target to a separate vessel for treatment.

It is thus an object of the present invention to provide a method of isolating and treating a particulate target, wherein the particulate target is a single animal cell or an aggregate thereof preferably located on a substrate, using an apparatus comprising
at least a capillary comprising a tip orifice and an internal cavity, said internal cavity comprising a treatment liquid,
a positioning element capable of positioning the tip orifice of the capillary,
a discharging/drawing element capable of causing a discharge of liquid from the capillary as well as causing a drawing of liquid into the capillary, wherein the discharged and/or drawn volume is selected in the range of 1 to 1000 nl,
characterized in that said method comprises the steps of:
   a. locating on the substrate the particulate target to be isolated and treated;
   b. positioning the tip orifice of the capillary such that the tip orifice of the capillary comes in proximity to the particulate target using the positioning element;
   c. discharging a sufficient volume of treatment liquid such as to essentially enclose the particulate target in the treatment liquid using the discharging/drawing element;
   d. drawing the particulate target enclosed in the treatment liquid into the internal cavity of the capillary,
   e. treating the particulate target within the internal cavity of the capillary comprising the treatment liquid.

### DESCRIPTION OF DRAWINGS

Preferred embodiments of the invention are described in the following with reference to the drawings, which are for the purpose of illustrating the present preferred embodiments of the invention and not for the purpose of limiting the same. In the drawings,

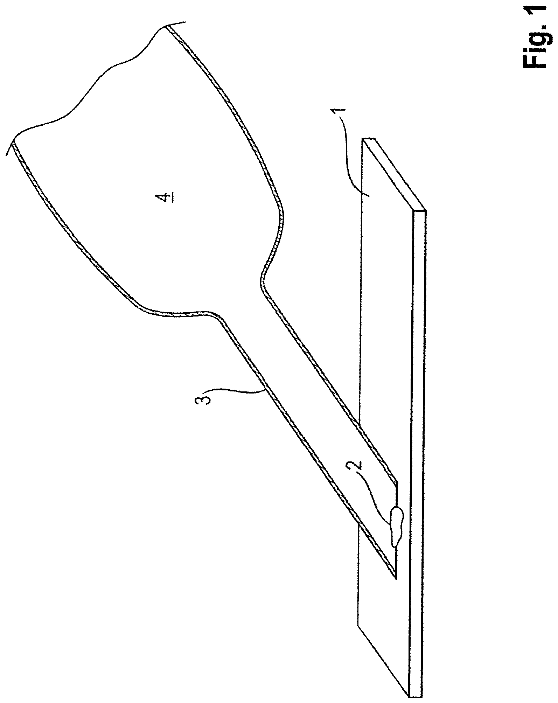

- Fig. 1: shows a capillary (3) having an internal cavity (4), and whose tip orifice is positioned in proximity of a particulate target (2) located on a substrate (1).

### DESCRIPTION OF PREFERRED EMBODIMENTS

It is thus an object of the present invention to provide a method of isolating and treating a particulate target, wherein the particulate target is a single animal cell or an aggregate thereof, using an apparatus comprising
at least a capillary comprising a tip orifice and an internal cavity, said internal cavity comprising a treatment liquid,
a positioning element capable of positioning the tip orifice of the capillary,
a discharging/drawing element capable of causing a discharge of liquid from the capillary as well as causing a drawing of liquid into the capillary, wherein the discharged and/or drawn volume is selected in the range of 1 to 1000 nl,
characterized in that said method comprises the steps of:
   a. locating the particulate target to be isolated and treated;
   b. positioning the tip orifice of the capillary such that the tip orifice of the capillary comes in proximity to the particulate target using the positioning element;
   c. discharging a volume of treatment liquid such as to enclose the particulate target in the treatment liquid using the discharging/drawing element;
   d. drawing the particulate target enclosed in the treatment liquid into the internal cavity of the capillary,
   e. treating the particulate target within the internal cavity of the capillary comprising the treatment liquid, and optionally
   f. introducing a neutralising liquid into the internal cavity of the capillary such as to neutralise the treatment liquid or discharging the particulate target into a recipient preferably comprising a neutralising liquid such as to neutralise the treatment liquid.

In the method according to the present invention, the particulate target may be a single animal cell such as circulating cells like a leukocyte, thrombocyte or erythrocytes, or cultured single animal cells such as non-adherent cells and adherent cells. Single animal cells which are of particular interest in the diagnostic context are circulating tumour cells (CTCs) which may be single CTCs or CTC clusters, which can for example be obtained by filtration from blood samples. In order to ease isolation of the cell, it may be labelled for example using fluorescent labels or dyes as is known in the art.

In the method according to the present invention, the particulate target is a single animal cell such as a eukaryotic or prokaryotic cell, or an aggregate thereof.

In a preferred embodiment of the method according to the present invention, the treatment liquid is not a growth medium. The treatment liquid may be a enzymatic buffer for a proteolytic treatment which can be used to dissociate aggregates of cultured animal cells or single cultured animal cells from the substrate on which they are being cultured. An example of such an enzymatic buffer is trypsin buffer.

In a preferred embodiment of the method according to the present invention, the treatment liquid is not a growth medium. The treatment liquid may be a cell lysis buffer or a cell dissociation buffer, for example when the particulate target is a cell which is to be genetically analysed. An example of such a cell lysis buffers are sodium dihydrogen phosphate / disodium hydrogen phosphate buffer, Tris-HCl buffer, and HEPES-NaOH buffer. In general, treating the particulate target, i.e. the animal cell, in the treatment liquid, i.e. in the lysis buffer, lasts of from 1 second to 10 minutes, preferably of from 5 seconds to 5 minutes.

In a preferred embodiment of the method according to the present invention, the substrate may be any suitable body capable of carrying the particulate target, such as for example a glass slide, a polymer membrane, a filter plate or filter membrane, or the bottom plate of a cell culture container. In the case where the particulate target is is a circulating tumor cell (CTC) the substrate can be a filter plate or filter membrane.

In a preferred embodiment of the method according to the present invention, the tip orifice of the capillary is positioned such that the orifice of the capillary comes at least within 20 µm, preferably within 10 µm, of the particulate target and/or wherein the particulate target has a size of from 100 nm to 100 µm, preferably of from 500 nm to 50 µm, more preferably of from 1 µm to 50 µm.

An apparatus that may be used in the method of isolating and treating a particulate target according to the present invention comprises at least a capillary comprising a tip orifice and an internal cavity, said internal cavity comprising a treatment liquid, a positioning element capable of positioning the tip orifice of the capillary, a discharging/drawing element capable of causing a discharge of treatment liquid from the capillary as well as causing a drawing of treatment liquid into the capillary.

Capillaries comprising a tip orifice and an internal cavity can be purchased commercially. While the diameter of the tip orifice is not specially limited since it must be chosen such that the particulate target can enter the capillary, in most cases the diameter of the tip orifice will be in the range of 1 µm to 1000 µm, more preferably of from 10 µm to 100 µm and even more preferably of from 10 µm to 50 µm. The diameter of the internal cavity is not specially limited, as long as the diameter of the internal cavity is at least equal to or larger than the diameter of the tip orifice. As an example a suitable diameter will be in the range of 100 µm to 5000 µm and preferably in the range of 100 µm to 1000 µm. The capillary may be formed from any suitable material such as glass, polymer or quartz.

In a preferred embodiment of the method according to the present invention, the capillary can further comprise one or more integrated monitoring area configured to allow monitoring of the treatment of a particulate target in the internal cavity of the capillary by monitoring means such as for example optical means such as a spectrometer. The integrated monitoring area corresponds to an area on the capillary which area is configured to allow the transmission of electromagnetic radiation having a predetermined wavelength or range of wavelengths, such as for example UV, IR or VIS radiation, across the integrated monitoring area and into the internal cavity and back. Preferably, the integrated monitoring area is configured to allow the transmission of preferably at least 75% or at least 80% of a radiation. The integrated monitoring area may be made from different materials, depending on the required wavelength of the electromagnetic radiation used. For instance, suitable materials are glass having an optical wavelength transmission ranging of from 340 to 2,500 nm, polymer having a wavelength transmission range of from 380 to 780 nm (VIS) and quartz having a wavelength transmission range of below 380 nm (UV). The one or more integrated monitoring area can be formed for example as two diametrally opposed integrated monitoring areas or as one continuous integrated monitoring area encircling the capillary thus forming a ring integrated monitoring area.

In a preferred embodiment of the method according to the present invention, drawing the particulate target enclosed in the treatment liquid into the internal cavity of the capillary is performed essentially immediately after enclosing the particulate target in the treatment liquid such that the treatment is essentially carried out in the internal cavity of the capillary. In particular in the case where the particulate target is a single animal cell or aggregate thereof, the drawing the particulate target enclosed in the treatment liquid into the internal cavity of the capillary is performed within 1 second after enclosing the particulate target in the treatment liquid.

In a preferred embodiment of the method according to the present invention, the apparatus can further comprise a monitoring element configured to monitor the treatment of a particulate target in the internal cavity of the capillary, preferably via the integrated monitoring area. The monitoring element preferably comprises a radiation source and a radiation detector. The radiation source may be a light source capable of emitting UV, IR or VIS light within defined range of wavelength whereas the radiation detector is capable of either detecting light within the same defined range of wavelength or for example, as is the case when the treatment of the particulate target in the internal cavity of the capillary is monitored by measuring fluorescence, detecting light of a corresponding range of emitted wavelength. In a preferred embodiment of the methods and apparatuses according to the present invention, the radiation source, the integrated monitoring area and the radiation detector are arranged along a common axis.

In a preferred embodiment of the method according to the present invention and in the case where the capillary further comprises an integrated monitoring area configured to allow monitoring of the treatment of a particulate target in the internal cavity of the capillary by optical means, the apparatus further comprises a control element configured to i) receive information indicative of the progress of the treatment of the particulate target in the internal cavity of the capillary from the monitoring element and to ii) bring about the discharge of the particulate target from the capillary when receiving information indicative of the completion of the treatment from the monitoring element by controlling the discharging/drawing element.

In a preferred embodiment of the method according to the present invention and in the case for example where the monitoring element comprises a radiation source and a radiation detector, the control unit is configured i) to receive information indicative of the progress of the treatment of the particulate target such as a cell in the internal cavity of the capillary in form of electrical or optical signals from both the radiation source and the radiation detector and, and ii) bring about the discharge of the particulate target such as a cell from the capillary receiving information indicative of the completion of the treatment from the monitoring element by controlling the discharging/drawing element.

In a preferred embodiment of the method according to the present invention, the apparatus further comprises a releasing element configured to release the particulate target from a substrate by for example ultrasonic pulse or a laser pulse. In the case where the releasing element facilitates the release of the particulate target by ultrasonic pulse, the releasing element may be an ultrasonic transducer needle or spatula, configured to be positioned in proximity to the particulate target via for example a second positioning element. In the case where the releasing element facilitates the release of the particulate target by laser pulse, the releasing element may be a laser emitting element that can be focussed in proximity to the particulate target via for example a second positioning element and which can deliver consecutive pulses of 0.1-1000 µJ, preferably of 1-100µJ and/or for about 1-100000 psec, preferably for about 1-10000 psec. The wavelength is preferably selected so that the particulate target is unaffected in its integrity, i.e. for the case of a cell is not killed. In one embodiment of the present invention, the releasing element may be a needle or spatula, without ultrasound or laser, formed from a suitable material such as polymer or metal.

In a preferred embodiment of the method according to the present invention, at least the tip orifice of the capillary and/or the internal cavity and/or the releasing element is further lined with a hydrophobic layer such as for example a polysiloxan layer or fluoropolymer layer or with a hydrophilic layer to enhance the wetting of the tip orifice of the capillary and/or the internal cavity and/or the releasing element.

In a preferred embodiment of the method according to the present invention, the tip portion and/or the longitudinal axis of the capillary is oriented in a normal direction with respect to a plane defined by the substrate or the tip portion and/or the longitudinal axis of the capillary is oriented in a non-normal direction with respect to a plane defined by the substrate and is preferably oriented of from 30 to 60 degrees with respect to the plane defined by the substrate.

### LIST OF REFERENCE SIGNS

| | | | |
|---|---|---|---|
| 1 | support | 3 | capillary |
| 2 | particulate target | 4 | internal cavity |

## Claims

1. A method of isolating and treating a particulate target (2) using an apparatus, wherein the particulate target (2) is a single animal cell or an aggregate thereof, comprising
at least a capillary (3) comprising a tip orifice and an internal cavity (4), said internal cavity (4) comprising a treatment liquid,
a positioning element capable of positioning the tip orifice of the capillary (3),
a discharging/drawing element capable of causing a discharge of treatment liquid from the capillary (3) as well as causing a drawing of treatment liquid into the capillary (3), wherein the discharged and/or drawn volume can be selected in the range of 1 to 1000 nl, **characterized in that** said method comprises the steps of:
a. locating the particulate target (2) to be isolated and treated
b. positioning the tip orifice of the capillary (3) such that the tip orifice of the capillary (3) comes in proximity to the particulate target (2), using the positioning element,
c. discharging of treatment liquid such as to enclose the particulate target (2) in the treatment liquid,
d. drawing the particulate target (2) enclosed in the treatment liquid into the internal cavity (4) of the capillary (3),
e. treating the particulate target (2) within the internal cavity (4) of the capillary (3) comprising the treatment liquid.

2. The method of isolating and treating a cell according to claim 2, wherein the treatment liquid is not a growth medium.

3. The method of isolating and treating a particulate target (2) according to claim 1 or 2, wherein the treatment liquid is a cell lysis buffer or a proteolytic treatment buffer.

4. The method of isolating and treating a particulate target (2) according to any preceding claim, wherein the orifice of the capillary is positioned such that the orifice of the capillary comes at least within 20 µm, preferably at least within 10 µm, of the particulate target (2) and/or wherein the particulate target (2) has a size of from 100 nm to 100 µm, preferably of from 500 nm to 50 µm and/or wherein the diameter of the tip orifice is in the range of 1 µm to 1000 µm, preferably of from 10 µm to 100 µm.

5. The method of isolating and treating a particulate target (2) according to any preceding claim, wherein the particulate target substrate is located on a substrate (1) preferably chosen from microscope slides, filter membranes such as CTC-filters or the bottom plate of a cell culturing container preferably containing cell culturing medium.

6. The method according to claim 3, wherein the treatment liquid is a cell lysis buffer and said method further comprises the steps of:
f. introducing a neutralising liquid into the internal cavity (4) of the capillary (3) such as to neutralise the cell lysis buffer or discharging a cell lysate into a recipient preferably comprising a neutralising liquid such as to neutralise the cell lysis buffer.

7. The method according to claim 3, wherein the treatment liquid is a proteolytic buffer and said method comprises the steps of:
f. introducing a neutralising liquid into the internal cavity (4) of the capillary (3) such as to neutralise the proteolytic buffer or discharging the proteolytically treated single animal cell or an aggregate thereof into a recipient preferably comprising a neutralising liquid such as to neutralise the proteolytic buffer.

## Patentansprüche

1. Verfahren zur Isolierung und Behandlung eines partikulären Zielobjekts (2) unter Verwendung einer Vorrichtung, wobei das partikuläre Zielobjekt (2) eine einzelne tierische Zelle oder ein Aggregat davon ist, aufweisend
- mindestens ein Kapillarrohr (3), welches eine Spitzenöffnung und einen inneren Hohlraum (4) aufweist, wobei der besagte innere Hohlraum (4) eine Behandlungsflüssigkeit enthält,
- ein Positionierungselement, welches fähig ist, die Spitzenöffnung des Kapillarrohrs (3) zu positionieren,
- ein Entleerungs-/Aufzieh-Element, welches fähig ist, eine Entleerung der Behandlungsflüssigkeit aus dem Kapillarrohr (3) zu bewirken, sowie ein Aufziehen von Behandlungsflüssigkeit in das Kapillarrohr (3) hinein zu bewirken, wobei die entleerte und/oder aufgezogene Menge im Bereich von 1 bis 1000 nl ausgewählt werden kann, **dadurch gekennzeichnet, dass** das besagte Verfahren die folgenden Schritte beinhaltet:
a. Lokalisieren des zu isolierenden und zu behandelnden partikulären Zielobjektes (2),
b. Positionieren der Spitzenöffnung des Kapillarrohrs (3), sodass die Spitzenöffnung (3) in die Nähe des partikulären Zielobjektes (2) zu liegen kommt, unter Verwendung des Positionierungselements,
c. Entleeren der Behandlungsflüssigkeit, sodass das partikuläre Zielobjekt (2) von der Behandlungsflüssigkeit umschlossen wird,
d. Aufziehen des von der Behandlungsflüssigkeit umschlossenen partikulären Zielobjekts (2) in den inneren Hohlraum (4) des Kapillarrohrs (3) hinein,
e. Behandeln des partikulären Zielobjekts (2) innerhalb des die Behandlungsflüssigkeit enthaltenden inneren Hohlraums (4) des Kapillarrohrs (3).

2. Verfahren zur Isolierung und Behandlung einer Zelle gemäss Anspruch 2, wobei die Behandlungsflüssigkeit kein Wachstumsmedium ist.

3. Verfahren zur Isolierung und Behandlung eines partikulären Zielobjektes (2) gemäss Anspruch 1 oder 2, wobei die Behandlungsflüssigkeit ein Zell-Lyse-Puffer oder ein proteolytischer Behandlungspuffer ist.

4. Verfahren zur Isolierung und Behandlung eines partikulären Zielobjektes (2) gemäss einem der vorhergehenden Ansprüche, wobei die Öffnung des Kapillarrohrs derart positioniert ist, dass die Öffnung des Kapillarrohrs mindestens in einen Bereich von 20 µm Entfernung, vorzugsweise mindestens in einen Bereich von 10 µm Entfernung zum partikulären Zielobjekt (2) kommt, und/oder wobei das partikuläre Zielobjekt (2) eine Grösse von 100 nm bis 100 µm aufweist, vorzugsweise von 500 nm bis 50 µm und/oder wobei der Durchmesser der Spitzenöffnung im Bereich von 1 µm bis 1000 µm liegt, vorzugsweise von 10 µm bis 100 µm.

5. Verfahren zur Isolierung und Behandlung eines partikulären Zielobjektes (2) gemäss einem der vorhergehenden Ansprüche, wobei das Substrat des Zielobjektes auf einem Substrat (1) angeordnet ist, welches vorzugsweise ausgewählt ist aus Objektträgern, Filtermembranen wie beispielsweise CTC Filtern, oder der Bodenplatte eines Zellkulturbehälters, welcher vorzugsweise Zellkulturmedium enthält.

6. Verfahren gemäss Anspruch 3, wobei die Behandlungsflüssigkeit ein Zell-Lyse-Puffer ist und wobei das besagte Verfahren ferner die folgenden Schritte beinhaltet:
f. Einführen einer neutralisierenden Flüssigkeit in den inneren Hohlraum (4) des Kapillarrohrs (3), zum Neutralisieren des Zell-Lyse-Puffers oder Entleeren eines Zell-Lysats in einen Behälter, welcher vorzugsweise eine neutralisierende Flüssigkeit zum Neutralisieren des Zell-Lyse-Puffers enthält.

7. Verfahren gemäss Anspruch 3, wobei die Behandlungsflüssigkeit ein proteolytischer Puffer ist und wobei das besagte Verfahren die folgenden Schritte beinhaltet:
f. Einführen einer neutralisierenden Flüssigkeit in den inneren Hohlraum (4) des Kapillarrohrs (3), zum Neutralisieren des proteolytischen Puffers oder Entleeren der proteolytisch behandelten einzelnen tierischen Zelle oder eines Aggregats davon in einen Behälter, welcher vorzugsweise eine neutralisierende Flüssigkeit zum Neutralisieren des proteolytischen Puffers enthält.

## Revendications

1. Un procédé d'isolement et de traitement d'une cible particulaire (2) à l'aide d'un appareil, dans lequel la cible particulaire (2) est une cellule animale individuelle ou un agrégat de celle-ci, comprenant
au moins un capillaire (3) comprenant un orifice de pointe et une cavité interne (4), ladite cavité interne (4) comprenant un liquide de traitement,
un élément de positionnement apte à positionner l'orifice de pointe du capillaire (3),
un élément de décharge/aspiration capable de provoquer une décharge de liquide de traitement du capillaire (3) ainsi que de provoquer une aspiration de liquide de traitement dans le capillaire (3), dans lequel ledit le volume déchargé/aspiré est choisi dans la plage de 1 à 1000 ni, **caractérisé en ce que** ledit procédé comprend les étapes consistant à:
a. localiser la cible particulaire (2) à isoler et à traiter
b. positionner l'orifice de pointe du capillaire (3) de telle sorte que l'orifice de pointe du capillaire (3) vient à proximité de la cible particulaire (2), à l'aide de l'élément de positionnement,
c. décharge du liquide de traitement de manière à enfermer la cible particulaire (2) dans le liquide de traitement,
d. aspirer la cible particulaire (2) enfermée dans le liquide de traitement dans la cavité interne (4) du capillaire (3),
e. traiter la cible particulaire (2) à l'intérieur de la cavité interne (4) du capillaire (3) comprenant le liquide de traitement.

2. Le procédé d'isolement et de traitement d'une cellule selon la revendication 2, dans lequel le liquide de traitement n'est pas un milieu de croissance.

3. Le procédé d'isolement et de traitement d'une cible particulaire (2) selon la revendication 1 ou 2, dans lequel le liquide de traitement est un tampon de lyse cellulaire ou un tampon de traitement protéolytique.

4. Le procédé d'isolement et de traitement d'une cible particulaire (2) selon l'une quelconque des revendications précédentes, dans lequel l'orifice du capillaire est positionné de telle sorte que l'orifice du capillaire se situe au moins à 20 um, de préférence au moins à 10 um, de la cible particulaire (2) et/ ou dans laquelle la cible particulaire (2) a une taille de 100 nm à 100 um, de préférence de 500 nm à 50 um et/ou dans lequel le diamètre de l'orifice de pointe est dans la plage de 1 um à 1000 um , de préférence de 10 um à 100 um .

5. Le procédé d'isolement et de traitement d'une cible particulaire (2) selon l'une quelconque des revendications précédentes, dans lequel la cible particulaire est située sur un substrat (1) de préférence choisi parmi les lames de microscope, les membranes filtrantes telles que les filtres CTC ou la plaque inférieure d'un récipient de culture cellulaire contenant de préférence du milieu de culture cellulaire.

6. Le procédé selon la revendication 3, dans lequel le liquide de traitement est un tampon de lyse cellulaire et ledit procédé comprend en outre les étapes consistant à:
f. introduire un liquide neutralisant dans la cavité interne (4) du capillaire (3) de manière à neutraliser le tampon de lyse cellulaire ou décharger le lysat cellulaire dans un récipient comprenant de préférence un liquide neutralisant de manière à neutraliser le tampon de lyse cellulaire.

7. Le procédé selon la revendication 3, dans lequel le liquide de traitement est un tampon protéolytique et ledit procédé comprend en outre les étapes consistant à:
f. introduire un liquide neutralisant dans la cavité interne (4) du capillaire (3) de manière à neutraliser le tampon protéolytique ou décharger la cellule individuelle traitée de manière protéolytique ou un agrégat de celle-ci dans un récipient comprenant de préférence un liquide neutralisant de manière à neutraliser le tampon protéolytique.
